# EUROPEAN PATENT SPECIFICATION

(11) **EP 3 626 272 B1**
(45) Date of publication and mention of the grant of the patent: **04.10.2023**
(21) Application number: 18801291.8
(22) Date of filing: 16.05.2018
(51) Int. Cl.: A61K 47/38, A61K 9/20, A61K 47/26, A61K 9/00

(54) **COMPOSITION FOR DISINTEGRATING TABLETS CONTAINING MICROFIBROUS CELLULOSE AND ACTIVE INGREDIENT**
ZUSAMMENSETZUNG ZUR ZERKLEINERUNG VON TABLETTEN MIT MIKROFASERCELLULOSE UND WIRKSTOFF
CELLULOSE MICROFIBREUSE, ET COMPOSITION POUR COMPRIMÉ SE DÉSINTÉGRANT À TENEUR EN PRINCIPES ACTIFS

(30) Priority: 18.05.2017 JP 2017098688
(43) Date of publication of application: 25.03.2020
(73) Proprietor: Daicel Corporation, Osaka-shi, Osaka 530-0011 (JP)
(72) Inventor: MATSUOKA, Mio, Himeji-shi Hyogo 671-1281 (JP); HASHIKAWA, Naohiro, Himeji-shi Hyogo 671-1281 (JP); HAMASAKI, Momoko, Himeji-shi Hyogo 671-1281 (JP); TAKIGAWA, Yoshihisa, Himeji-shi Hyogo 671-1281 (JP)
(74) Representative: J A Kemp LLP
(86) International application number: PCT/JP2018/018871
(87) International publication number: WO 2018/212220

(56) References cited:
- EP-A1- 3 135 300
- WO-A1-2015/163135
- WO-A1-2017/002803
- US-A1- 2002 061 335
- KOLAKOVIC, R. et al.: "Spray-dried cellulose nanofibers as novel tablet excipient", AAPS Pharma. Sci. Tech., vol. 12, no. 4, December 2011 (2011-12), pages 1366-1373, XP019985244,

## Description

### Technical Field

The present invention relates to a composition for a disintegrating tablet, comprising various kinds of active ingredients such as a medicinal, a nutritional ingredient, a supplement and a food component; and a disintegrator component consisting of only micro-fibrillated cellulose; and to various kinds of disintegrating tablets comprising said composition.

### Background Art

Cellulose that is produced from a vegetable fiber and having a fiber diameter (a short diameter) or thickness of from about a few nm to a few µm has been generally known as "fine-fibrillated cellulose" or "micro-fibrillated cellulose." The production examples and its structure, properties and functions are described in Patent Literature (PTL) 1 and PTL 2 cited below.

In the fine- or micro-fibrillated cellulose, a surface area has been increased, hydrophilic property that is the original characteristics of cellulose has been significantly strengthened, and a three-dimensional network has been formed, without deteriorating basic properties such as physical and chemical stabilities of a starting material of cellulose. As a result, when it is formulated into goods in a paste or cream shape, it will show a water-retaining (syneresis-preventing) property and a form-retaining property due to the interaction with water and oil droplets, fine particles, etc. It is also utilized to modify goods in a jelly form, for example, to increase their strength.

Accordingly, the above cellulose has been widely used in various applications, for example, as a binder for powder and fibrous materials, a paper strong agent in papermaking, a thickening agent for improving food texture of foods, a humectant for water-retaining of foods, a filter aid for alcoholic beverage and the like.

As an application example of the micro-fibrillated cellulose, PTL 3 describes a gelly composition comprising a water-dispersible complex comprising the micro-fibrillated cellulose and a hydrophilic polymer that is soluble in warm water in a particular ratio; a gelling agent; and water in a particular ratio. It describes that the composition has properties to inhibit denaturation of proteins and precipitation of water-insoluble components during heating or warming treatment and to give a good food texture.

PTL 4 describes a gelling agent comprising a highly dispersible cellulose complex comprising the micro-fibrillated cellulose, a water-soluble polymer and hydrophilic substance in a particular ratio; and a particular kind of polysaccharide in a particular ratio. It describes that the agent is characterized as being superior in disintegration and dispersion in water when compared to a conventional highly dispersible cellulose complex, so that it can be used in industrial and practical dispersing conditions.

Thus, the micro-fibrillated cellulose is used as a one component in the gelly composition and gelling agent disclosed in PTL 3 and 4. Furthermore, the hydrophilic polymer is an essential component for the water-dispersible complex of PTL 3, and the water-soluble polymer is an essential component for the highly dispersible cellulose complex of PTL 4.

PTL 5 describes an easily-flowable powder composition prepared by dispersing highly hygroscopic pharmaceuticals, cosmetics or food in micro-fibrillated cellulose as an invention for the purpose of the provision of hygroscopic powder that is easy to handle. Specifically, it discloses an easily-flowable powder composition comprising 50-70 wt% of extract from yeast or Kakkon-to (cinnamon) powder. It also discloses that a tablet made by tableting the composition has excellent formability (moldability). However, it never discloses anything about disintegrability of the tablet.

PTL 6 describes a binder made of cellulose having specific surface area of about 5m²/g or more such as bacterial cellulose and micro-fibrillated cellulose for the purpose of provision of a binder providing sufficient tablet hardness even with a low forming pressure, a composition comprising the binder, and a method for the production of a solid formulation by using the composition. It also describes that tablet hardness of the solid formulation has been improved in examples. However, the solid formulation does not substantially contain any active ingredient such as the medicinal ingredient. Furthermore, it never refers to disintegrability of the produced tablet.

PTL 7 describes a composition for a disintegrating tablet, which comprises micro-fibrillated cellulose. However, it is characterized by comprising other disintegrator(s) in addition to the micro-fibrillated cellulose. As a result, while the tablet hardness of the disintegrating tablets comprising the composition increases due to their synergistic effect, disintegration time in water of the tablets becomes shorter.

### Related Arts

### Patent Literatures

PTL 1: JP-A-Sho56(1981)-100801
PTL 2: JP-A-2009-203559
PTL 3: JP-A-2004-283135
PTL 4: JP-A-2006-290972
PTL 5: JP-A-Sho 61(1986)-236731
PTL 6: JP-A-Hei 11(1999)-60507
PTL 7: WO 2015/163135A1
PTL 8: JP-A-2007-231438

### Summary of Invention

### Problems to be solved by the Invention

The prior arts disclose no example of a composition for a disintegrating tablet, utilizing excellent properties of the micro-fibrillated cellulose, and various kinds of disintegrating tablets comprising the composition.

Especially, no disintegrating tablet for pharmaceuticals, Chinese medicine and various kinds of foods such as supplemental foods, nutrition function foods and health foods has been provided so far, which comprises various kinds of active ingredients such as the medicinal, the nutritional ingredient, the supplement and the food component at such a high concentration as about 80-90 wt%.

Accordingly, an object of the present invention is to solve such technical problems as mentioned above, and to provide various kinds of disintegrating tablets having a variety of purposes or applications such as pharmaceuticals, Chinese medicine and various kinds of foods, which comprise various kinds of the active ingredients such as the medicinal, the nutritional ingredient, the supplement and the food component at the high concentration.

### Means to Solve the Problem

The present inventors have earnestly studied and found that a tablet having excellent formability and disinterability can be produced using a disintegrator consisting of the micro-fibrillated cellulose only, leading to the completion of the present invention.

Thus, the present invention relates to the following aspects.

### [Aspect 1]

A composition for a disintegrating tablet, comprising a disintegrator component consisting of micro-fibrillated cellulose only, and an active ingredient, wherein the composition does not comprise any disintegrators other than the micro-fibrillated cellulose.

### [Aspect 2]

The composition for a disintegrating tablet according to Aspect 1, consisting of the micro-fibrillated cellulose and the active ingredient.

### [Aspect 3]

The composition for a disintegrating tablet according to Aspect 1 or 2,
wherein the micro-fibrillated cellulose has an average fiber length of 0.01~2 mm and an average fiber diameter of 0.001 ~1µm.

### [Aspect 4]

The composition for a disintegrating tablet according to any one of Aspects 1-3, comprising 80-90 wt% of the active ingredient.

### [Aspect 5]

The composition for a disintegrating tablet according to any one of Aspects 1-4, further comprising an excipient.

### [Aspect 6]

The composition for a disintegrating tablet according to Aspect 5, comprising sugars or sugar alcohols as the excipient.

### [Aspect 7]

A disintegrating tablet for pharmaceuticals or foods, comprising the composition for a disintegrating tablet according to any one of Aspects 1-6, wherein the tablet does not comprise any disintegrators other than the micro-fibrillated cellulose.

### [Aspect 8]

The disintegrating tablet according to Aspect 7, which has tablet hardness of from 10 to 200(N), and disintegration time in water of 6 min or less, and/or [disintegration time in water(D)]/[tablet hardness(H)] is 30(sec/N) or less.

### [Aspect 9]

A method for the production of the composition for a disintegrating tablet according to any one of Aspects 1-6, comprising:
injecting a hot air into a lower part of powder consisting of components other than the micro-fibrillated cellulose in a fluidized-bed granulator; and
spraying dispersion liquid (slurry) of the micro-fibrillated cellulose on an upper part of the powder while fluidizing the powder.

### Advantages of Invention

Since it is not necessary to comprise any disintegrator component other than a relatively small amount of the micro-fibrillated cellulose in the present composition, the active ingredient can be comprised at such a high concentration as about 80-90 wt%. Furthermore, it is possible to produce the disintegrating tablet having an excellent tablet hardness, and disintegrability or disversibility, and showing an excellent formability in the production thereof, by using the above composition.

### Description of Embodiments of the Invention

The present invention relates to the composition for a disintegrating tablet, comprising a disintegrator component consisting of micro-fibrillated cellulose only, and an active ingredient; and to the integrating tablet comprising the composition. Thus, both the composition and the tablet according to the present invention are characterized by not comprising any substance known as the disintegrator to those skilled in the art other than the micro-fibrillated cellulose.

The "active ingredient" means any substance that will show any activity or function such as nutritional, physiological or pharmaceutical one as the use or purpose of the disintegrating tablet in a subject such as human who has taken the disintegrating tablet. There is no limit with respect to the substance on its composition, raw material, origin, means of acquisition and the like. Thus, the active ingredient includes various types of the substance such as a natural product, natural extract, chemically synthesized product, pure chemicals, mixture, composition, etc.

An example of the composition for a disintegrating tablet according to the present invention is one consisting of the micro-fibrillated cellulose and the active ingredient.

Any cellulose conventionally known as the "fine-fibrillated cellulose" or "micro-fibrillated cellulose" can be used as the "micro-fibrillated cellulose" in the present invention.

As already mentioned, the micro-fibrillated cellulose is generally produced from the vegetable fiber and having the fiber diameter (the short diameter) or thickness of from about a few nm to a few µm. The surface area of the the micro-fibrillated cellulose has been increased, its hydrophilic property that is the original characteristics of cellulose has been significantly strengthened, and its three-dimensional network has been formed, without deteriorating the basic properties such physical and chemical stabilities of the starting material of cellulose.

Preferable examples of the micro-fibrillated cellulose comprised in the composition for the disintegrating tablet according to the present invention include fiber assembly that has an average fiber length of 0.01~2 mm and an average fiber diameter of 0.001 ~1µm, preferably of 0.01 ~ 0.1µm (PTL 2). For example, such micro-fibrillated cellulose is commercially available with a trade name of "CELISH" series (a solid content of 10~35 % in water) with various grades (an average fiber diameter of 0.01 ~ 0.1µm) from Daicel FineChem Ltd.

A dry material of the micro-fibrillated cellulose may be directly obtained in a dry state by any method known in the art, such as by directly pulverizing cellulose fiber such as any crystalline cellulose and/or powdered cellulose known to those skilled in the art in a dry state with a ball mill (PTL 1). Alternatively, the dry material of the micro-fibrillated cellulose may be obtained by subjecting the micro-fibrillated cellulose suspended in water, which was prepared by micro-fibrillation of water-dispersion of the cellulose fiber with a high-pressure homogenizer, to a solvent displacement stage, and removing the solvent in a drying stage, followed by pulverization in a pulverizing stage (PTL 2). Alternatively, a wet material of the micro-fibrillated cellulose may be prepared by means of the method described in PTL 8.

The crystalline cellulose and powdered cellulose is a white water-insoluble powdery substance obtained by partially depolymerizing α-cellulose, which is obtained from fibrous plants, with acids, followed by purification. The crystalline cellulose has no taste, and, since the substance is chemically inactive, it does not change even when being mixed with medicaments. Therefore, the crystalline cellulose has been used for purposes of a pharmaceutical additive, in particular, an auxiliary excipient, binder, disintegrator or the like for preparing tablets. In addition, the crystalline cellulose has been used as an emulsification stabilizer or the like for cosmetics, dairy products, etc. besides the additive for pharmaceuticals.

As typical examples of the crystalline cellulose, commercially-available products such as "Avicel" (FMC Corporation), "CEOLUS" (Asahi Kasei Chemicals Corp.), and "VIVAPUR" (RETTENMAIER) can be mentioned. As typical examples of the powdered cellulose, commercially-available products such as KC Flock (NIPPON PAPER Chemicals CO., LTD) and ARBOCEL (RETTENMAIER) and Solka Flock (Kimura Sangyo Co., Ltd.)

The composition for a disintegrating tablet according to the present invention may comprise sugars or sugar alcohols as the excipient in order to provide the excellent tablet hardness and disintegrability with the disintegrating tablet.

The sugars or sugar alcohols include mannitol, erythritol, xylitol, trehalose, lactose, maltose, maltitol, glucose, sucrose, fructose, mannose, isomalt, paratinose and sorbitol. Moreover, as preferable examples thereof, mannitol, erythritol, xylitol, trehalose, and lactose can be mentioned. As the sugars or sugar alcohols, two or more types of compounds properly selected from these compounds can also be used.

Furthermore, in addition to the above-described components, various types of optional components (ingredients) known to those skilled in the art (except any substance known as the disintegrator to those skilled in the art other than the micro-fibrillated cellulose) may properly be added and mixed into the composition for the disintegrating tablet of the present invention, for the purpose of adjusting various characteristics such as the disintegrating force, binding force and ease in taking the tablet, without impairing the effects of the present invention by the above-described components. As examples of such components, auxiliary excipients, fluidizing agents, sweetening agents, corrigents, flavoring agents and coloring agents can be mentioned.

The above crystalline cellulose and/or powdered cellulose, and inorganic excipients may be used as the auxiliary excipients . Examples of the inorganic excipients include light anhydrous silicic acid, silicon dioxide hydrate, anhydrous calcium phosphate, anhydrous calcium hydrogenphosphate, aluminum metasilicate, calcium silicate, magnesium silicate, and magnesium oxide.

Examples of the disintegrator components known to those skilled in the art that are not used in the present invention include carmellose, crospovidone, croscarmellose sodium, low substituted hydroxypropylcellulose, carboxymethylcellulose calcium, starch such as corn starch, potato starch, waxy corn starch, α-starch or partially α-starch, and processed starch such as starch sodium glycolate and hydroxypropyl starch. Additionally, crospovidone is a common name for a cross-linked polymer of 1-vinyl-2-pyrrolidone, and croscarmellose sodium is a common name for a cross-linked product of carboxymethylcellulose sodium.

An amount or content of each component comprised in the composition for the disintegrating tablet of the present invention can properly be determined by a person skilled in the art, depending on, for example, the type of the component, the type and purpose of the disintegrating tablet. In general, relative to the total weight of the disintegrative particulate composition, an amount of the active ingredient is within such a relatively high range as 80% to 90% by weight, an amount of the micro-fibrillated cellulose (in terms of a dry weight) is within a range of 0.1% to 20% by weight, an amount of the sugars or sugar alcohols as the excipient is within a range of 0% to 19.9% by weight, and the auxiliary excipient is within a range of 0% to 19.9% by weight.

The composition for the disintegrating tablet according to the present invention may be produced by any method or means known to those skilled in the art.

For examples, the composition for the disintegrating tablet may be produced by mixing each of the components comprised in the composition all together.

Alternatively, it may be produced by various granulation processes. As there is no limitation on granulation means, a dry granulation process and a wet granulation process may be used to produce the composition.

The dry granulation process comprises the steps of mixing each powder of the components comprised in the composition for the disintegrating tablet optionally with an appropriate binder and the like, breaking the resulting mixture into small bulks with a high pressure, and appropriately crushing and granulating them. Examples of the dry granulation process include crushing granulation and roll-compressing method.

The wet granulation process is a method in which each component is dispersed in the presence of water, and the dispersion is dried to form complexes. As specific examples of the wet granulation process, spray methods such as spray drying, tumbling granulation, agitation granulation and fluidized-bed granulation; the freeze-drying method; kneading granulation, and the like can be mentioned, and the composition can be produced by any of these methods known to a person skilled in the art.

The composition for the disintegrating tablet according to the present invention may be produced by one wet granulation step using all of the components comprised therein together, or by adding and mixing each component in the plural wet granulation steps.

A person skilled in the art can properly determine which one or two types of the components comprised in the disintegrative particulate composition shall be used in the above plural wet granulation steps, depending on their types, amounts, etc.

Furthermore, a person skilled in the art can properly determine various conditions in the above plural wet granulation steps, such as the spraying speed, the supply air temperature, the exhaust temperature, and the air supply rate, depending on types or amounts of components, etc.

In each of the above granulation step, as a medium for the spray liquid, a solvent acceptable in pharmaceuticals or foods, such as water, ethanol, methanol or acetone, can be mentioned. Alternatively, as the spray liquid, for example, an aqueous suspension comprising less than 10% of the component (s) of the composition can be mentioned.

As shown in the examples, the composition for the disintegrating tablet according to the present invention is produced by:
injecting a hot air into a lower part of powder consisting of components such as the active ingredient other than the micro-fibrillated cellulose, (the powder comprising all the components of the composition except the micro-fibrillated cellulose) in a fluidized-bed granulator; and
spraying dispersion liquid (slurry) of the micro-fibrillated cellulose on an upper part of the powder while fluidizing the powder.

Each of the above components that may be optionally comprised in the composition for the disintegrating tablet according to the present invention may be optionally added in any of the above granulation steps. Alternatively, the above optional components may be added and mixed in an additionally-provided wet granulation step.

It is preferable that the composition for the disintegrating tablet of the present invention produced by the above wet granulation process has the following physical properties:
(1) an average particle size of 50 to 500 microns; and
(2) a water content of 0.1% to 15% by weight.

The physical properties are measured by using the following methods and conditions.

The average particle size: 5 g of the composition for the disintegrating tablet is subjected to a measurement with a Φ75 mm electromagnetically vibrating sieve device (Type "M-3T", Tsutsui Scientific Instruments Co., Ltd.).

The water content: 5 g of the composition for the disintegrating tablet is subjected to a measurement using a halogen water content measuring device (Type "MX-50", A&D Company, Limited).

The present invention also relates to the disintegrating tablet for the various uses, comprising the above composition for the disintegrating tablet. Examples of the disintegrating tablet include disintegrating tablets for pharmaceuticals, and various kinds of foods such as drink, supplemental foods, nutrition function foods and health foods. There is no specific limitation in taing manner or form of the disintegrating tablet.

Specifically, the disintegrating tablet according to the present invention include any orally disintegrating tablets known for those skilled in the art, which will gradually disintegrate in gastrointestine after being orally ingested, in addition to an "orally disintegrating tablet" that will rapidly disintegrate in an oral cavity without water. Furthermore, in addition to the directly and orally-taking manner where the tablet is directly ingested orally, the tablet may be orally ingested in an indirectly and orally taking manner where the tablet is first mixed with medium such as water and hot water so as to form an optional condition such as dispersion, sol and the like, and it is then ingested.

The active ingredients comprised in the composition for the disintegrating tablet may be therefore optionally selected in accordance with the purposes, use or applications of the disintegrating tablet.

For example, the composition for the disintegrating tablet for foods may optionally comprise as the active ingredient various nutritional components such as proteins, carbohydrates, lipids and minerals; various vitamins and their derivatives; raw materials for health foods such as extracts originated from microorganisms, plants, animals and the like, etc.

The composition for the disintegrating tablet for pharmaceuticals may optionally comprise as the active ingredient any medicinal ingredients known for those skilled in the art, such as, for example, agents affecting each organ such as the central nervous system, peripheral nervous system, a sensory organ, a circulatory organ, a respiratory organ and a digestive organ and an urogenital organ; hormone drug; agents affecting metabolism such as a vitamin drug, an analeptic, an agent affecting blood and body fluid; agents affecting the function of tissue and cell such as an agent activating cellular function, an agent affecting tumors, an radioactive medicine, an anti-allergic agent; medicines based on a medical prescription relating to herbal medicines and Chinese medicines; antibiotics; agents for chemotherapy, biological drug; agents for pathogenic organisms such as parasites; agents for formulation use, diagnosis, and public health.

As the composition for the disintegrating tablet according to the present invention already comprise the active ingredients as mentioned above, it can be directly formulated so as to easily produce the integrating tablet. Alternatively, the disintegrating tablet according to the present invention may further comprise any other components except the disintegrator in addition to the above composition, depending on, for example, the application and purpose of the disintegrating tablet, for example, designated or existing additives according to Food Sanitation Law, Art. 10; and other components acceptable as a food component (a food additive) listed in a list of general additives for food and drink, such as acidulants, sweeteners, excipients, surfactants, lubricants, sweeteners, corrigents, flavoring agents, colorants, and stabilizing agents; and any ingredients described in "Japanese Pharmaceutical Excipients Directory" (YAKUJI NIPPO LIMITED) or the Japanese Pharmacopoeia.

As long as the desired effects by the present invention are obtained, the amount or content of the composition for the disintegrating tablet or other optional components are not limited, and can properly be determined by those skilled in the art. The disintegrating tablet can be formulated by any method known for those skilled in the art such as tableting. There is no particular limitation on the size, shape and form of the tablet, and can be properly determined, depending on the above application, taking manner and form of the disintegrating tablet.

The disintegrating tablet has the excellent tablet hardness and disintegrability since it comprises the composition for the disintegrating tablet of the present invention. As shown by the examples, when it is produced at tablet compression forces of 2 to 30 kN, it is characterized by having a hardness of 10 to 200 N, preferably 15 to 200 N, more preferably 20 to 200 N; and by having a disintegration time in water of 40 seconds or less, preferably 30 seconds or less in the case of the directly and orally taking tablet (the orally disintegrating tablet); and having a disintegration time in water of 6 minutes or less, preferably 5 minutes or less, more preferably 4 minutes or less in the case of the other disintegrating tablets such as one that will disintegrate in the gastrointestine,. Furthermore, with respect to the [disintegration time in water (D)]/ [tablet hardness (H)] (sec/N), the orally disintegrating tablet has 0.005 - 2 (sec/N), preferably 0.005 - 0.75(sec/N); and the other disintegrating tablets have 0.005 - 15 (sec/N), preferably 0.005 - 6(sec/N).

In the case of the disintegrating tablet that is taken in the indirectly and orally taking manner where the tablet is first mixed with medium so as to form an optional condition such as dispersion, sol and the like, and it is then ingested, the tablet has a disintegration time in water of 5 minutes or less, preferably one minute or less; and [disintegration time in water(D)]/[tablet hardness(H)] (sec/N) of 0.005-30 (sec/N), preferably 0.005-5(sec/N), more preferably 0.005-3(sec/N).

In addition, contents of all related art documents cited in the present specification are incorporated herein by reference.

Hereinafter, the present invention will more specifically be described with reference to Examples. However, the present invention is not considered to be limited to the Examples.

### [Evaluation on hardness and disintegrability tests]

The results about the tablet hardness, disintegrability in water and [disintegration time in water(D)]/[tablet hardness(H)] (sec/N) of each tablet obtained in Examples and Comparative Examples are shown in Tables 1-4.

Hardness: a hardness (N) was measured with a digital Kiya hardness tester (Fujiwara Scientific Company Co., Ltd.).

Disintegration time in water: a disintegration time in water was measured with a disintegration tester (NT-400, TOYAMA SANGYO CO., LTD.) in accordance with the method described in the Japanese Pharmacopoeia provided that an auxiliary disk was not used.

The measurements for the hardness and disintegration time were repeated three times and twice, respectively, and average values thereof were regarded as measurement results.

In the Comparative Examples, when the tablet hardness is less than 10 (N), the pharase "not measured due to the hardness <10(N)" is described in the corresponding column in Tables

### Examples

### [Example 1]

### [Production of disintegrative particulate composition 1]

270 g of glycyrrhiza (powdered glycyrrhiza, MATSUURA YAKUGYO CO., LTD. ) was charged to a fluidized-bed granulator (FL-LABO, Freund Corporation) and granulated by spraying 600 g of 5% suspension of wet material of micro-fibrillated cellulose ("CELISH FD200L", Daicel FineChem Ltd.) in water at a rate of 4g/min onto the granulator, so as to obtain disintegrative particulate composition 1.

### [Production of disintegrating tablet 1]

The resulting disintegrative particulate composition 1 was subjected to tableting at a tablet compression force described in Table 1 with a simple tableting machine (HANDTAB-100, ICHIHASHI-SEIKI Co., Ltd.) to thereby obtain an angled-corner flat tablet having a diameter of 8.0 mm and a weight of 200 mg.

### [Example 2]

### [Production of disintegrative particulate composition 2]

270 g of glycyrrhiza (powdered glycyrrhiza, MATSUURA YAKUGYO CO., LTD. ) was charged to a fluidized-bed granulator (FL-LABO, Freund Corporation) and granulated by spraying 600 g of 5% suspension of wet material (7%:viscosity, 960mPa·S) of the micro-fibrillated cellulose, which was prepared from crystalline cellulose ("CEOLUS PH-102", Asahi Kasei Chemicals Corp.) by the method described in JP-A-2007-231438, in water at a rate of 4g/min onto the granulator, so as to obtain disintegrative particulate composition 2.

### [Production of disintegrating tablet 2]

The resulting disintegrative particulate composition 2 was subjected to tableting at a tablet compression force described in Table 1 with a simple tableting machine (HANDTAB-100, ICHIHASHI-SEIKI Co., Ltd.) to thereby obtain an angled-corner flat tablet having a diameter of 8.0 mm and a weight of 200 mg.

### [Example 3]

### [Production of disintegrative particulate composition 3]

270 g of glycyrrhiza (powdered glycyrrhiza, MATSUURA YAKUGYO CO., LTD. ) was charged to a fluidized-bed granulator (FL-LABO, Freund Corporation) and granulated by spraying 600 g of 5% suspension of wet material (7%:viscosity, 670mPa·S) of the micro-fibrillated cellulose, which was prepared from crystalline cellulose ("CEOLUS KG-802", Asahi Kasei Chemicals Corp.) by the method described in JP-A-2007-231438, in water at a rate of 4g/min onto the granulator, so as to obtain disintegrative particulate composition 3.

### [Production of disintegrating tablet 3]

The resulting disintegrative particulate composition 3 was subjected to tableting at a tablet compression force described in Table 1 with a simple tableting machine (HANDTAB-100, ICHIHASHI-SEIKI Co., Ltd.) to thereby obtain an angled-corner flat tablet having a diameter of 8.0 mm and a weight of 200 mg.

### [Comparative Example 1]

Glycyrrhiza (powdered glycyrrhiza, MATSUURA YAKUGYO CO., LTD.) was subjected to tableting at a tablet compression force described in Table 1 with a simple tableting machine (HANDTAB-100, ICHIHASHI-SEIKI Co., Ltd.) to thereby obtain an angled-corner flat tablet having a diameter of 8.0 mm and a weight of 200 mg. It could not be formulated into a tablet when it was subjected to tableting at a tablet compression force of 3kN.

### [Comparative Example 2]

90 parts by weight of glycyrrhiza (powdered glycyrrhiza, MATSUURA YAKUGYO CO., LTD. ) was mixed with 10 parts by weight of partially α-starch ("PCS PC-10", Asahi Kasei Chemicals Corp.), and then subjected to tableting at a tablet compression force described in Table 1 with a simple tableting machine (HANDTAB-100, ICHIHASHI-SEIKI Co., Ltd.) to thereby obtain an angled-corner flat tablet having a diameter of 8.0 mm and a weight of 200 mg. It could not be formulated into a tablet when it was subjected to tableting at a tablet compression force of 3kN.

### [Example 4]

### [Production of disintegrative particulate composition 4]

90 g of peony (peony powder, MATSUURA YAKUGYO CO., LTD.) was charged to a fluidized-bed granulator (FL-LABO, Freund Corporation) and granulated by spraying 200 g of 5% suspension of wet material of micro-fibrillated cellulose ("CELISH FD200L", Daicel FineChem Ltd.) in water at a rate of 2.5g/min onto the granulator, so as to obtain disintegrative particulate composition 4.

### [Production of disintegrating tablet 4]

The resulting disintegrative particulate composition 4 was subjected to tableting at a tablet compression force described in Table 1 with a simple tableting machine (HANDTAB-100, ICHIHASHI-SEIKI Co., Ltd.) to thereby obtain an angled-corner flat tablet having a diameter of 8.0 mm and a weight of 200 mg.

### [Comparative Example 3]

Peony (peony powder, MATSUURA YAKUGYO CO., LTD.) was subjected to tableting at a tablet compression force described in Table 1 with a simple tableting machine (HANDTAB-100, ICHIHASHI-SEIKI Co., Ltd.) to thereby obtain an angled-corner flat tablet having a diameter of 8.0 mm and a weight of 200 mg. It could not be formulated into a tablet when it was subjected to tableting at a tablet compression force of 3kN.

### [Example 5]

### [Production of disintegrative particulate composition 5]

90 g of N-acetylglucosamine (Marine Sweet YSK, Yaizu Suisankagaku Industry Co.,Ltd.) was charged to a fluidized-bed granulator (FL-LABO, Freund Corporation) and granulated by spraying 200 g of 5% suspension of wet material of micro-fibrillated cellulose ("CELISH FD200L", Daicel FineChem Ltd.) in water at a rate of 2.5 g/min onto the granulator, so as to obtain disintegrative particulate composition 5.

### [Production of disintegrating tablet 5]

The resulting disintegrative particulate composition 5 was subjected to tableting at a tablet compression force described in Table 2 with a simple tableting machine (HANDTAB-100, ICHIHASHI-SEIKI Co., Ltd.) to thereby obtain an angled-corner flat tablet having a diameter of 12.0 mm and a weight of 500 mg.

### [Comparative Example 4]

N-acetylglucosamine (Marine Sweet YSK, Yaizu Suisankagaku Industry Co.,Ltd.)was subjected to tableting at a tablet compression force described in Table 2 with a simple tableting machine (HANDTAB-100, ICHIHASHI-SEIKI Co., Ltd.) to thereby obtain an angled-corner flat tablet having a diameter of 12.0 mm and a weight of 500 mg.

### [Example 6]

### [Production of disintegrative particulate composition 6]

90 g of glucosamine (KOYO Glucosamine SC, KOYO CHEMICAL CO.,LTD.) was charged to a fluidized-bed granulator (FL-LABO, Freund Corporation) and granulated by spraying 200 g of 5% suspension of wet material of micro-fibrillated cellulose ( "CELISH FD200L", Daicel FineChem Ltd.) in water at a rate of 2.5 g/min onto the granulator, so as to obtain disintegrative particulate composition 6.

### [Production of disintegrating tablet 6]

The resulting disintegrative particulate composition 6 was subjected to tableting at a tablet compression force described in Table 2 with a simple tableting machine (HANDTAB-100, ICHIHASHI-SEIKI Co., Ltd.) to thereby obtain an angled-corner flat tablet having a diameter of 12.0 mm and a weight of 500 mg.

### [Example 7]

### [Production of disintegrative particulate composition 7]

80 g of glucosamine (KOYO Glucosamine SC, KOYO CHEMICAL CO.,LTD.) was charged to a fluidized-bed granulator (FL-LABO, Freund Corporation) and granulated by spraying 400 g of 5% suspension of wet material of micro-fibrillated cellulose ("CELISH FD200L", Daicel FineChem Ltd.) in water at a rate of 2.5 g/min onto the granulator, so as to obtain disintegrative particulate composition 7.

### [Production of disintegrating tablet 7]

The resulting disintegrative particulate composition 7 was subjected to tableting at a tablet compression force described in Table 2 with a simple tableting machine (HANDTAB-100, ICHIHASHI-SEIKI Co., Ltd.) to thereby obtain an angled-corner flat tablet having a diameter of 12.0 mm and a weight of 500 mg.

### [Example 8]

### [Production of disintegrative particulate composition 8]

90 g of glucosamine (KOYO Glucosamine SC, KOYO CHEMICAL CO.,LTD.) was charged to a fluidized-bed granulator (FL-LABO, Freund Corporation) and granulated by spraying 200 g of 5% suspension of wet material (7%:viscosity, 960mPa·S) of the micro-fibrillated cellulose, which was prepared from crystalline cellulose ("CEOLUS PH-102", Asahi Kasei Chemicals Corp.) by the method described in JP-A-2007-231438, in water at a rate of 4g/min onto the granulator, so as to obtain disintegrative particulate composition 8.

### [Production of disintegrating tablet 8]

The resulting disintegrative particulate composition 8 was subjected to tableting at a tablet compression force described in Table 2 with a simple tableting machine (HANDTAB-100, ICHIHASHI-SEIKI Co., Ltd.) to thereby obtain an angled-corner flat tablet having a diameter of 12.0 mm and a weight of 500 mg.

### [Example 9]

### [Production of disintegrative particulate composition 9]

90 g of glucosamine (KOYO Glucosamine SC, KOYO CHEMICAL CO.,LTD.) was charged to a fluidized-bed granulator (FL-LABO, Freund Corporation) and granulated by spraying 200 g of 5% suspension of wet material (7%:viscosity, 670mPa·S) of the micro-fibrillated cellulose, which was prepared from crystalline cellulose ("CEOLUS KG-802", Asahi Kasei Chemicals Corp.) by the method described in JP-A-2007-231438, in water at a rate of 4g/min onto the granulator, so as to obtain disintegrative particulate composition 9.

### [Production of disintegrating tablet 9]

The resulting disintegrative particulate composition 9 was subjected to tableting at a tablet compression force described in Table 2 with a simple tableting machine (HANDTAB-100, ICHIHASHI-SEIKI Co., Ltd.) to thereby obtain an angled-corner flat tablet having a diameter of 12.0 mm and a weight of 500 mg.

### [Comparative Example 5]

Although glucosamine (KOYO Glucosamine SC, KOYO CHEMICAL CO.,LTD.) was subjected to tableting at a tablet compression force described in Table 2 with a simple tableting machine (HANDTAB-100, ICHIHASHI-SEIKI Co., Ltd.), it could not be formulated into a tablet

### [Example 10]

### [Production of disintegrative particulate composition 10]

89.1 g of β-cryptoxanthin (CRP-015, DAICEL CORPORATION) and 0.9 g of micronized powder (Sylopage 720, FUJI SILYSIA CHEMICAL LTD.) were charged to a fluidized-bed granulator (FL-LABO, Freund Corporation) and granulated by spraying 200 g of 5% suspension of wet material of micro-fibrillated cellulose ("CELISH FD200L", Daicel FineChem Ltd.) in water at a rate of 2.5g/min onto the granulator, so as to obtain disintegrative particulate composition 10.

### [Production of disintegrating tablet 10]

The resulting disintegrative particulate composition 10 was subjected to tableting at a tablet compression force described in Table 2 with a simple tableting machine (HANDTAB-100, ICHIHASHI-SEIKI Co., Ltd.) to thereby obtain an angled-corner flat tablet having a diameter of 12.0 mm and a weight of 500 mg.

### [Comparative Example 6]

β-cryptoxanthin (CRP-015, DAICEL CORPORATION) was subjected to tableting at a tablet compression force described in Table 2 with a simple tableting machine (HANDTAB-100, ICHIHASHI-SEIKI Co., Ltd.) to thereby obtain an angled-corner flat tablet having a diameter of 12.0 mm and a weight of 500 mg.

### [Example 11]

### [Production of disintegrative particulate composition 11]

90 g of Sparassis crispa (HNP-100, DAICEL CORPORATION) was charged to a fluidized-bed granulator (FL-LABO, Freund Corporation) and granulated by spraying 200 g of 5% suspension of wet material of micro-fibrillated cellulose ("CELISH FD200L", Daicel FineChem Ltd.) in water at a rate of 2.5g/min onto the granulator, so as to obtain disintegrative particulate composition 11.

### [Production of disintegrating tablet 11]

The resulting disintegrative particulate composition 11 was subjected to tableting at a tablet compression force described in Table 2 with a simple tableting machine (HANDTAB-100, ICHIHASHI-SEIKI Co., Ltd.) to thereby obtain an angled-corner flat tablet having a diameter of 12.0 mm and a weight of 500 mg.

### [Example 12]

### [Production of disintegrative particulate composition 12]

80 g of Sparassis crispa (HNP-100, DAICEL CORPORATION) was charged to a fluidized-bed granulator (FL-LABO, Freund Corporation) and granulated by spraying 400 g of 5% suspension of wet material of micro-fibrillated cellulose ("CELISH FD200L", Daicel FineChem Ltd.) in water at a rate of 2.5g/min onto the granulator, so as to obtain disintegrative particulate composition 12.

### [Production of disintegrating tablet 12]

The resulting disintegrative particulate composition 12 was subjected to tableting at a tablet compression force described in Table 2 with a simple tableting machine (HANDTAB-100, ICHIHASHI-SEIKI Co., Ltd.) to thereby obtain an angled-corner flat tablet having a diameter of 12.0 mm and a weight of 500 mg.

### [Comparative Example 7]

Sparassis crispa (HNP-100, DAICEL CORPORATION) was subjected to tableting at a tablet compression force described in Table 2 with a simple tableting machine (HANDTAB-100, ICHIHASHI-SEIKI Co., Ltd.) to thereby obtain an angled-corner flat tablet having a diameter of 12.0 mm and a weight of 500 mg. It could not be formulated into a tablet when it was subjected to tableting at a tablet compression force of 14kN.

### [Example 13]

### [Production of disintegrative particulate composition 13]

270 g of green tea powder (domestic green tea powder, SEISHIN ENTERPRISE Co.,Ltd.) was charged to a fluidized-bed granulator (FL-LABO, Freund Corporation) and granulated by spraying 600 g of 5% suspension of wet material of micro-fibrillated cellulose ("CELISH FD200L", Daicel FineChem Ltd.) in water at a rate of 4g/min onto the granulator, so as to obtain disintegrative particulate composition 13.

### [Production of disintegrating tablet 13]

The resulting disintegrative particulate composition 13 was subjected to tableting at a tablet compression force described in Table 3 with a simple tableting machine (HANDTAB-100, ICHIHASHI-SEIKI Co., Ltd.) to thereby obtain an angled-corner flat tablet having a diameter of 8.0 mm and a weight of 200 mg.

### [Example 14]

### [Production of disintegrative particulate composition 14]

240 g of green tea powder (domestic green tea powder, SEISHIN ENTERPRISE Co.,Ltd.) was charged to a fluidized-bed granulator (FL-LABO, Freund Corporation) and granulated by spraying 1200 g of 5% suspension of wet material of micro-fibrillated cellulose ("CELISH FD200L", Daicel FineChem Ltd.) in water at a rate of 6g/min onto the granulator, so as to obtain disintegrative particulate composition 14.

### [Production of disintegrating tablet 14]

The resulting disintegrative particulate composition 14 was subjected to tableting at a tablet compression force described in Table 3 with a simple tableting machine (HANDTAB-100, ICHIHASHI-SEIKI Co., Ltd.) to thereby obtain an angled-corner flat tablet having a diameter of 8.0 mm and a weight of 200 mg.

### [Comparative Example 8]

Green tea powder (domestic green tea powder, SEISHIN ENTERPRISE Co.,Ltd.) was subjected to tableting at a tablet compression force described in Table 3 with a simple tableting machine (HANDTAB-100, ICHIHASHI-SEIKI Co., Ltd.) to thereby obtain an angled-corner flat tablet having a diameter of 8.0 mm and a weight of 200 mg. It could not be formulated into a tablet when it was subjected to tableting at a tablet compression force of 5kN.

### [Example 15]

### [Production of disintegrative particulate composition 15]

240 g of green barley powder (domestic green barley powder produced in Fukuoka prefecture, SEISHIN ENTERPRISE Co.,Ltd.) was charged to a fluidized-bed granulator (FL-LABO, Freund Corporation) and granulated by spraying 1200 g of 5% suspension of wet material of micro-fibrillated cellulose ("CELISH FD200L", Daicel FineChem Ltd.) in water at a rate of 6g/min onto the granulator, so as to obtain disintegrative particulate composition 15.

### [Production of disintegrating tablet 15]

The resulting disintegrative particulate composition 15 was subjected to tableting at a tablet compression force described in Table 3 with a simple tableting machine (HANDTAB-100, ICHIHASHI-SEIKI Co., Ltd.) to thereby obtain an angled-corner flat tablet having a diameter of 14.0 mm and a weight of 500 mg.

### [Comparative Example 9]

Green barley powder (domestic green barley powder produced in Fukuoka prefecture, SEISHIN ENTERPRISE Co.,Ltd.) was subjected to tableting at a tablet compression force described in Table 3 with a simple tableting machine (HANDTAB-100, ICHIHASHI-SEIKI Co., Ltd.) to thereby obtain an angled-corner flat tablet having a diameter of 14.0 mm and a weight of 500 mg. It could not be formulated into a tablet when it was subjected to tableting at a tablet compression force of 7kN.

### [Example 16]

### [Production of disintegrative particulate composition 16]

240 g of kale powder (domestic kale powder produced in Miyazaki prefecture, SEISHIN ENTERPRISE Co.,Ltd.) was charged to a fluidized-bed granulator (FL-LABO, Freund Corporation) and granulated by spraying 1200 g of 5% suspension of wet material of micro-fibrillated cellulose ("CELISH FD200L", Daicel FineChem Ltd.) in water at a rate of 6g/min onto the granulator, so as to obtain disintegrative particulate composition 16.

### [Production of disintegrating tablet 16]

The resulting disintegrative particulate composition 16 was subjected to tableting at a tablet compression force described in Table 3 with a simple tableting machine (HANDTAB-100, ICHIHASHI-SEIKI Co., Ltd.) to thereby obtain an angled-corner flat tablet having a diameter of 14.0 mm and a weight of 500 mg.

### [Comparative Example 10]

Kale powder (domestic green barley powder produced in Miyazaki prefecture, SEISHIN ENTERPRISE Co.,Ltd.) was subjected to tableting at a tablet compression force described in Table 3 with a simple tableting machine (HANDTAB-100, ICHIHASHI-SEIKI Co., Ltd.), but it could not be formulated into a tablet.

**[Table 1]**

| | Example 1 | | Example 2 | |
|---|---|---|---|---|
| Tablet Compression Force (kN) | 3 | 5 | 3 | 5 |
| Tablet Hardness (N) | 64 | 106 | 78 | 119 |
| Disintegration Time in Water(s) | 88 | 236 | 134 | 280 |
| D/H (sec/N) | 1. 4 | 2. 2 | 1. 7 | 2. 4 |
| | | | | |

| | Example 3 | | Example 4 | |
|---|---|---|---|---|
| Tablet Compression Force (kN) | 3 | 5 | 3 | 5 |
| Tablet Hardness (N) | 75 | 117 | 49 | 98 |
| Disintegration Time in Water(s) | 95 | 231 | 82 | 330 |
| D/H (sec/N) | 1. 3 | 2. 0 | 1. 7 | 3. 4 |
| | | | | |

| | Compar Ex. ative 1 | | Compar Ex. ative .2 | |
|---|---|---|---|---|
| Tablet Compression Force (kN) | 3 | 6 | 3 | 5 |
| Tablet Hardness (N) | not measured due to the hardness <10(N) | 37 | not measured due to the hardness <10(N) | 14 |
| Disintegration Time in Water(s) | | 745 | | 291 |
| D/H (sec/N) | | 20. 2 | | 21. 1 |
| | | | | |

| | Comparat ive Ex.3 | | | |
|---|---|---|---|---|
| Tablet Compression Force (kN) | 3 | 10 | | |
| Tablet Hardness (N) | not measured due to the hardness <10(N) | 34 | | |
| Disintegration Time in Water(s) | | 149 | | |
| D/H (sec/N) | | 4. 4 | | |

**[Table 2]**

| | | | Ex. 5 | Example 6 | | Ex.7 |
|---|---|---|---|---|---|---|
| Tablet Compress ion Force (kN) | | | 4 | 8 | 10 | 4 |
| Tablet Har dness (N) | | | 69 | 52 | 67 | 49 |
| Disintegration T ime in Water (s) | | | 23 | 299 | 256 | 278 |
| D/H (s ec/N) | | | 0. 3 | 5. 7 | 3. 8 | 5. 7 |
| | | | | | | |

| | | | Example 8 | | Example 9 | |
|---|---|---|---|---|---|---|
| Tablet Compress ion Force (kN) | | | 8 | 10 | 8 | 10 |
| Tablet Har dness (N) | | | 52 | 69 | 47 | 49 |
| Disintegration T ime in Water (s) | | | 73 | 67 | 89 | 105 |
| D/H (s ec/N) | | | 1. 4 | 1.0 | 1.9 | 2. 1 |
| | | | | | | |
| | | | Ex. 10 | Example 11 | | Ex. 12 |
| Tablet Compress ion Force (kN) | | | 10 | 14 | 16 | 9 |
| Tablet Har dness (N) | | | 24 | 53 | 75 | 75 |
| Disintegration T ime in Water (s) | | | 37 | 123 | 180 | 56 |
| D/H (s ec/N) | | | 1. 5 | 2. 3 | 2. 4 | 0. 7 |
| | | | | | | |

| | Comparative Example 4 | Com Ex parative ample 5 | | Comparative Example 6 | Compa Exam arative ple 7 | |
|---|---|---|---|---|---|---|
| Tablet Compression Force (kN) | 4 | 8 | | 10 | 14 | 20 |
| Tablet Hardness (N) | 18 | not measured due to the hardness <10(N) | 21 | | not measured due to the hardness <10(N) | 13 |
| Disintegration Time in Water (s) | 44 | | 710 | | | 766 |
| D/H (sec/N) | 2. 4 | | 33. 8 | | | 60. 8 |

**[Table 3]**

| | Ex. 13 | Example 14 | | | Ex.15 |
|---|---|---|---|---|---|
| Tablet Compression Force (kN) | 12 | 5 | | 12 | 7 |
| Tablet Hardness (N) | 33 | 31 | | 60 | 26 |
| Disintegration Time in Water(s) | 155 | 21 | | 165 | 49 |
| D/H (sec/N) | 4. 7 | 0. 7 | | 2. 7 | 1. 9 |
| | | | | | |

| | Example 15 | | Example 16 | | |
|---|---|---|---|---|---|
| Tablet Compression Force (kN) | 9 | | 15 | | |
| Tablet Hardness (N) | 39 | | 30 | | |
| Disintegration Time in Water(s) | 104 | | 58 | | |
| D/H (sec/N) | 2. 7 | | 1. 9 | | |

| | Comparative Ex.8 | | Comparative Ex. 9 | | |
|---|---|---|---|---|---|
| Tablet Compression Force (kN) | 5 | 12 | 7 | 20 | |
| Tablet Hardness (N) | not measured due to the hardness <10(N) | 14 | not measured due to the hardness <10(N) | 20 | |
| Disintegration Time in Water(s) | | 1008 | | 1719 | |
| D/H (sec/N) | | 72. 6 | | 86. 0 | |
| | | | | | |

| | Comparative Ex. 10 | | | | |
|---|---|---|---|---|---|
| Tablet Compression Force (kN) | 15 | | | | |
| Tablet Hardness (N) | not measured due to the hardness <10(N) | | | | |
| Disintegration Time in Water(s) | | | | | |
| D/H (sec/N) | | | | | |

The results shown in Tables 1 -3 demonstrate that the tablets produced in Examples 1-16 by using the granules (the disintegrative particulate composition according the present invention) produced by the method comprising the wet granulation step using the micro-fibrillated cellulose slurry have more excellent tablet hardness, disintegrability and [disintegration time in water (D)] / [tablet hardness (H)] (sec/N) than the tablets not processed by the above method (Comparative Examples 1-10)

### [Example 17]

### [Production of disintegrative particulate composition 17]

90 g of glucosamine (KOYO Glucosamine SC, KOYO CHEMICAL CO.,LTD.) was charged to a fluidized-bed granulator (FL-LABO, Freund Corporation) and granulated by spraying 200 g of 5% suspension of wet material of micro-fibrillated cellulose ( "CELISH FD200L", Daicel FineChem Ltd.) in water at a rate of 2.5 g/min onto the granulator, so as to obtain disintegrative particulate composition 17 (the same as disintegrative particulate composition 6).

### [Production of disintegrating tablet 17]

70 parts by weight of the resulting disintegrative particulate composition 17 was mixed with 30 parts by weight of D-mannitol (PEARLITOL 50C, Roquette Pharma), and subjected to tableting at a tablet compression force described in Table 4 with a simple tableting machine (HANDTAB-100, ICHIHASHI-SEIKI Co., Ltd.) to thereby obtain an angled-corner flat tablet having a diameter of 12.0 mm and a weight of 500 mg.

### [Comparative Example 11]

70 parts by weight of glucosamine (KOYO Glucosamine SC, KOYO CHEMICAL CO.,LTD.) was mixed with 30 parts by weight of D-mannitol (PEARLITOL 50C, Roquette Pharma), and subjected to tableting at a tablet compression force described in Table 4 with a simple tableting machine (HANDTAB-100, ICHIHASHI-SEIKI Co., Ltd.) to thereby obtain an angled-corner flat tablet having a diameter of 12.0 mm and a weight of 500 mg.

### [Example 18]

### [Production of disintegrative particulate composition 18]

270 g of glycyrrhiza (powdered glycyrrhiza, MATSUURA YAKUGYO CO., LTD. ) was charged to a fluidized-bed granulator (FL-LABO, Freund Corporation) and granulated by spraying 600 g of 5% suspension of wet material of micro-fibrillated cellulose ("CELISH FD200L", Daicel FineChem Ltd.) in water at a rate of 4g/min onto the granulator, so as to obtain disintegrative particulate composition 18 (the same as disintegrative particulate composition 1).

### [Production of disintegrating tablet 18]

90 parts by weight of the resulting disintegrative particulate composition 18 was mixed with 10 parts by weight of D-mannitol (PEARLITOL 50C, Roquette Pharma), and subjected to tableting at a tablet compression force described in Table 4 with a simple tableting machine (HANDTAB-100, ICHIHASHI-SEIKI Co., Ltd.) to thereby obtain an angled-corner flat tablet having a diameter of 8.0 mm and a weight of 200 mg.

### [Comparative Example 12]

90 parts by weight of glycyrrhiza (powdered glycyrrhiza, MATSUURA YAKUGYO CO., LTD. ) was mixed with 10 parts by weight of D-mannitol (PEARLITOL 50C, Roquette Pharma), and subjected to tableting at a tablet compression force described in Table 4 with a simple tableting machine (HANDTAB-100, ICHIHASHI-SEIKI Co., Ltd.) to thereby obtain an angled-corner flat tablet having a diameter of 8.0 mm and a weight of 200 mg.

**[Table 4]**

| | Example 17 | | | Example 18 | | |
|---|---|---|---|---|---|---|
| Tablet Compression Force (kN) | 10 | 15 | | 3 | 5 | |
| Tablet Hardness (N) | 30 | 43 | | 43 | 75 | |
| Disintegration Time in Water(s) | 15 | 21 | | 41 | 150 | |
| D/H (sec/N) | 0. 5 | 0. 5 | | 0. 9 | 2. 0 | |
| | | | | | | |

| | Comparative Ex. 11 | | Comparative Ex.12 | | | |
|---|---|---|---|---|---|---|
| Tablet Compression Force (kN) | 15 | | 3 | | | 5 |
| Tablet Hardness (N) | not measured | | not | | | 13 |
| Disintegration Time in Water(s) | | | due to the hardness <10(N) | measured due to the hardness <10(N) | 309 | |
| D/H (sec/N) | | | | | 23. 7 | |

The results shown in Table 4 demonstrate that the granules (the disintegrative particulate composition according the present invention) produced in Examples 17 and 18 by the method comprising the wet granulation step using the micro-fibrillated cellulose slurry, even when it was further mixed with the excipient, show more excellent disintegrability than the tablets comprising the excipient but not processed by the above granulation step (Comparative Examples 11 and 12).

### [Example 19]

### [Production of disintegrating tablet 19]

70 parts by weight of the resulting disintegrative particulate composition 6 was mixed with 30 parts by weight of anhydrous calcium phosphate (Taihei Chemical Industrial Co., Ltd.), and subjected to tableting at a tablet compression force described in Table 5 with a simple tableting machine (HANDTAB-100, ICHIHASHI-SEIKI Co., Ltd.) to thereby obtain an angled-corner flat tablet having a diameter of 12.0 mm and a weight of 500 mg.

### [Example 20]

### [Production of disintegrating tablet 20]

70 parts by weight of the resulting disintegrative particulate composition 1 was mixed with 30 parts by weight of crystalline cellulose (CEOLUS PH-101, Asahi Kasei Chemicals Corp.), and subjected to tableting at a tablet compression force described in Table 5 with a simple tableting machine (HANDTAB-100, ICHIHASHI-SEIKI Co., Ltd.) to thereby obtain an angled-corner flat tablet having a diameter of 8.0 mm and a weight of 200 mg.

### [Example 21]

### [Production of disintegrating tablet 21]

70 parts by weight of the resulting disintegrative particulate composition 1 was mixed with 30 parts by weight of anhydrous calcium phosphate (Taihei Chemical Industrial Co., Ltd.), and subjected to tableting at a tablet compression force described in Table 5 with a simple tableting machine (HANDTAB-100, ICHIHASHI-SEIKI Co., Ltd.) to thereby obtain an angled-corner flat tablet having a diameter of 8.0 mm and a weight of 200 mg.

**[Table 5]**

| | | | | Ex. 19 | Ex. 20 | Ex. 21 |
|---|---|---|---|---|---|---|
| Tablet Compression Force (kN) | 10 | 15 | 3 | 5 | 3 | 5 |
| Tablet Hardness (N) | 42 | 55 | 60 | 97 | 46 | 73 |
| Disintegration Time in Water(s) | 110 | 208 | 69 | 184 | 47 | 161 |
| D/H (sec/N) | 2.6 | 3.8 | 1.1 | 1.9 | 1.0 | 2.2 |

The results shown in Table 5 demonstrate that the granules (the disintegrative particulate composition according the present invention) produced in Example 19 by the method comprising the wet granulation step using the micro-fibrillated cellulose slurry, even when it was further mixed with the auxiliary excipient, show more excellent disintegrability and [disintegration time in water (D)] / [tablet hardness (H)] (sec/N) than the granules in the corresponding Example 6 that comprise the same active ingredient of glucosamine but not the auxiliary excipient.

Similarly, the results shown in Table 5 demonstrate that the granules (the disintegrative particulate composition according the present invention) produced in Examples 20 and 21 by the method comprising the wet granulation step using the micro-fibrillated cellulose slurry, even when it was further mixed with the auxiliary excipient, show comparative to or more excellent disintegrability and [disintegration time in water (D)] / [tablet hardness (H)] (sec/N) than the granules in the corresponding Example 1 that comprise the same active ingredient of glycyrrhiza but not the auxiliary excipient.

### [Comparative Example 13]

70 parts by weight of glucosamine (KOYO Glucosamine SC, KOYO CHEMICAL CO.,LTD.) was mixed with 30 parts by weight of anhydrous calcium phosphate (Taihei Chemical Industrial Co., Ltd.), and subjected to tableting at a tablet compression force described in Table 6 with a simple tableting machine (HANDTAB-100, ICHIHASHI-SEIKI Co., Ltd.) to thereby obtain an angled-corner flat tablet having a diameter of 12.0 mm and a weight of 500 mg.

### [Comparative Example 14]

70 parts by weight of glycyrrhiza (powdered glycyrrhiza, MATSUURA YAKUGYO CO., LTD.) was mixed with 30 parts by weight of crystalline cellulose (CEOLUS PH-101, Asahi Kasei Chemicals Corp.), and subjected to tableting at a tablet compression force described in Table 6 with a simple tableting machine (HANDTAB-100, ICHIHASHI-SEIKI Co., Ltd.) to thereby obtain an angled-corner flat tablet having a diameter of 8.0 mm and a weight of 200 mg.

### [Comparative Example 15]

70 parts by weight of glycyrrhiza (powdered glycyrrhiza, MATSUURA YAKUGYO CO., LTD.) was mixed with 30 parts by weight of anhydrous calcium phosphate (Taihei Chemical Industrial Co., Ltd.), and subjected to tableting at a tablet compression force described in Table 6 with a simple tableting machine (HANDTAB-100, ICHIHASHI-SEIKI Co., Ltd.) to thereby obtain an angled-corner flat tablet having a diameter of 8.0 mm and a weight of 200 mg.

**[Table 6]**

| | Comparative Ex.13 | | Comparative Ex.14 | | Comparative Ex.15 | |
|---|---|---|---|---|---|---|
| Tablet Compression Force (kN) | 10 | 15 | 3 | 5 | 3 | 5 |
| Tablet Hardness (N) | not measured due to the hardness <10 (N) | | not measured due to the hardness <10(N) | 16 | not measured due to the hardness <10(N) | 17 |
| Disintegration Time in Water(s) | | | | 410 | | 215 |
| D/H (sec/N) | | | | 25.7 | | 12.4 |

Comparison between the results shown in Tables 5 and 6 demonstrate that the granules (the disintegrative particulate composition according the present invention) produced in Examples 19, 20 and 21 by the method comprising the wet granulation step using the micro-fibrillated cellulose slurry show more excellent disintegrability, tablet hardness and

[disintegration time in water (D)] / [tablet hardness (H)] (sec/N) than the tablets in the corresponding Comparative Examples 13, 14 and 15 that were produced only by the addition of the auxiliary excipient without using any granulation step.

### [Comparative Example 16]

219 g of D-mannitol (Mannit P, Mitsubishi Shoji Foodtech Co., Ltd.) and 60g of corn starch (Cornstarch White W-4P, JAPAN CORN STARCH CO. , LTD.) were charged to a fluidized-bed granulator (FL-LABO, Freund Corporation) and granulated by spraying 420 g of 5% suspension of wet material of micro-fibrillated cellulose ( "CELISH FD200L", Daicel FineChem Ltd.) in water at a rate of 12 g/min onto the granulator, so as to obtain disintegrative particulate composition.

### [Production of comparative disintegrating tablet 16]

90 parts by weight of N-acetylglucosamine (Marine Sweet YSK, Yaizu Suisankagaku Industry Co.,Ltd.) and 10 parts by weight of the resulting disintegrative particulate composition obtained in Comparative Example 16 were mixed, and subjected to tableting at a tablet compression force described in Table 7 with a simple tableting machine (HANDTAB-100, ICHIHASHI-SEIKI Co. , Ltd.) to thereby obtain an angled-corner flat tablet having a diameter of 12.0 mm and a weight of 500 mg.

### [Comparative Example 17]

90 parts by weight of glucosamine (KOYO Glucosamine SC, KOYO CHEMICAL CO., LTD.) and 10 parts by weight of the resulting disintegrative particulate composition obtained in Comparative Example 16 were mixed, and subjected to tableting at a tablet compression force described in Table 7 with a simple tableting machine (HANDTAB-100, ICHIHASHI-SEIKI Co., Ltd.) , but it could not be formulated into a tablet.

### [Comparative Example 18]

90 parts by weight of β-cryptoxanthin (CRP-015, DAICEL CORPORATION) and 10 parts by weight of the resulting disintegrative particulate composition obtained in Comparative Example 16 were mixed, and subjected to tableting at a tablet compression force described in Table 7 with a simple tableting machine (HANDTAB-100, ICHIHASHI-SEIKI Co., Ltd.) to thereby obtain an angled-corner flat tablet having a diameter of 12.0 mm and a weight of 500 mg.

### [Comparative Example 19]

80 parts by weight of Sparassis crispa (HNP-100, DAICEL CORPORATION) and 20 parts by weight of the resulting disintegrative particulate composition obtained in Comparative Example 16 were mixed, and subjected to tableting at a tablet compression force described in Table 7 with a simple tableting machine (HANDTAB-100, ICHIHASHI-SEIKI Co., \Ltd.) , but it could not be formulated into a tablet.

### [Comparative Example 20]

80 parts by weight of green tea powder (domestic green tea powder, SEISHIN ENTERPRISE Co.,Ltd.) and 20 parts by weight of the resulting disintegrative particulate composition obtained in Comparative Example 16 were mixed, and subjected to tableting at a tablet compression force described in Table 7 with a simple tableting machine (HANDTAB-100, ICHIHASHI-SEIKI Co., Ltd.) to thereby obtain an angled-corner flat tablet having a diameter of 8.0 mm and a weight of 200 mg.

### [Comparative Example 21]

80 parts by weight of Green barley powder (domestic green barley powder produced in Fukuoka prefecture, SEISHIN ENTERPRISE Co.,Ltd.) and 20 parts by weight of the resulting disintegrative particulate composition obtained in Comparative Example 16 were mixed, and subjected to tableting at a tablet compression force described in Table 7 with a simple tableting machine (HANDTAB-100, ICHIHASHI-SEIKI Co., Ltd.) to thereby obtain an angled-corner flat tablet having a diameter of 14.0 mm and a weight of 500 mg.

### [Comparative Example 22]

80 parts by weight of kale powder (domestic kale powder produced in Miyazaki prefecture, SEISHIN ENTERPRISE Co.,Ltd.) and 20 parts by weight of the resulting disintegrative particulate composition obtained in Comparative Example 16 were mixed, and subjected to tableting at a tablet compression force described in Table 7 with a simple tableting machine (HANDTAB-100, ICHIHASHI-SEIKI Co., Ltd.), but it could not be formulated into a tablet.

**[Table 7]**

| | Comparative Ex.16 | Comparative Ex.17 | Comparative Ex.18 | Comparative Ex. 19 |
|---|---|---|---|---|
| Tablet Compression Force (kN) | 18 | 20 | 20 | 20 |
| Tablet Hardness (N) | 68 | not measured due to the hardness <10(N) | 24 | not measured due to the hardness <10(N) |
| Disintegration Time in Water (s) | 50 | | 273 | |
| D/H (sec/N) | 0.7 | | 11.5 | |

| | Comparative Ex.20 | Comparative Ex.21 | Comparative Ex.22 | |
|---|---|---|---|---|
| Tablet Compression Force (kN) | 14 | 20 | 20 | |
| Tablet Hardness (N) | 17 | 32 | not measured due to the hardness <10(N) | |
| Disintegration Time in Water(s) | 180 | 1116 | | |
| D/H (sec/N) | 10.4 | 34.9 | | |

Comparison between the results of Comparative Examples 16-22 shown in Table 7, and the results of Example 5; Examples 6, 7 and 17; Example 10; Examples 11 and 12; Examples 13 and 14; Example 15; and Example 16, which correspond to each of the above Comparative Examples, respectively, demonstrate that the tablets using the granules (the disintegrative particulate composition according the present invention) produced by the method comprising the wet granulation step using as the disintegrator component the micro-fibrillated cellulose only have more excellent disintegrability, tablet hardness and [disintegration time in water (D)] / [tablet hardness (H)] (sec/N) than the tablets produced by the method comprising the wet granulation step using other disintegrator component in addition to the micro-fibrillated cellulose.

Incidentally, the Examples except Example 17 and Example 18 are different from the corresponding Comparative Examples 16-22 in that the said Examples do not comprise the excipient. However, when Examples 6 and 7, and Example 1 are compared to the related Example 17 and Example 18 comprising the excipient (D-Mannitol), respectively, it is clear that at least [disintegration time in water(D)]/[tablet hardness(H)] (sec/N) of the disintegrative particulate composition would be improved by the addition of the excipient. Accordingly, the superiority of use of the integrator component consisting only of the micro-fibrillated cellulose is obvious.

### Industrial Applicability

The present invention significantly contributes to research and development of orally-disintegrating tablets having excellent tablet hardness and disintegrability.

## Claims

1. A composition for a disintegrating tablet, comprising a disintegrator component consisting of only micro-fibrillated cellulose, and an active ingredient, wherein the composition does not comprise any disintegrators other than the micro-fibrillated cellulose.

2. The composition for a disintegrating tablet according to Claim 1, further comprising an excipient.

3. The composition for a disintegrating tablet according to Claim 2, comprising sugars or sugar alcohols as the excipient.

4. The composition for a disintegrating tablet according to Claim 1, consisting of the micro-fibrillated cellulose and the active ingredient.

5. The composition for a disintegrating tablet according to any one of Claims 1-4, wherein the micro-fibrillated cellulose has an average fiber length of 0.01~2 mm and an average fiber diameter of 0.001 ~1µm.

6. The composition for a disintegrating tablet according to any one of Claims 1-5, comprising 80-90 wt% of the active ingredient.

7. A disintegrating tablet for pharmaceuticals or foods, comprising the composition for a disintegrating tablet according to any one of Claims 1-6, wherein the tablet does not comprise any disintegrators other than the micro-fibrillated cellulose.

8. The disintegrating tablet according to Claim 7, which has tablet hardness of from 10 to 200(N), and disintegration time in water of 6 min or less, and/or [disintegration time in water(D)]/[tablet hardness(H)] is 30(sec/N) or less.

9. A method for the production of the composition for a disintegrating tablet according to any one of Claims 1-6, comprising:
injecting a hot air into a lower part of powder consisting of components other than the micro-fibrillated cellulose in a fluidized-bed granulator; and
spraying dispersion liquid (slurry) of the micro-fibrillated cellulose on an upper part of the powder while fluidizing the powder.

## Patentansprüche

1. Zusammensetzung für eine Zerfallstablette, umfassend eine Zerfallskomponente, die nur aus mikrofibrillierter Cellulose besteht, und einen Wirkstoff, wobei die Zusammensetzung außer der mikrofibrillierten Cellulose keine anderen Zerfallskomponenten umfasst.

2. Zusammensetzung für eine Zerfallstablette nach Anspruch 1, die ferner einen Hilfsstoff umfasst.

3. Zusammensetzung für eine Zerfallstablette nach Anspruch 2, die Zucker oder Zuckeralkohole als Hilfsstoff umfasst.

4. Zusammensetzung für eine Zerfallstablette nach Anspruch 1, die aus der mikrofibrillierten Cellulose und dem Wirkstoff besteht.

5. Zusammensetzung für eine Zerfallstablette nach einem der Ansprüche 1 bis 4, wobei die mikrofibrillierte Cellulose eine durchschnittliche Faserlänge von 0,01 bis 2 mm und einen durchschnittlichen Faserdurchmesser von 0,001 bis 1µm aufweist.

6. Zusammensetzung für eine Zerfallstablette nach einem der Ansprüche 1 bis 5, die 80 bis 90 Gew.-% des Wirkstoffs umfasst.

7. Zerfallstablette für Pharmazeutika oder Lebensmittel, umfassend die Zusammensetzung für eine Zerfallstablette nach einem der Ansprüche 1 bis 6, wobei die Tablette außer der mikrofibrillierten Cellulose keine anderen Zerfallshilfsstoffe umfasst.

8. Zerfallstablette nach Anspruch 7, die eine Tablettenhärte von 10 bis 200(N) und eine Zerfallszeit in Wasser von 6 min oder weniger aufweist, und/oder [Zerfallszeit in Wasser(D)] / [Tablettenhärte(H)] 30 (sec/N) oder weniger beträgt.

9. Verfahren zur Herstellung der Zusammensetzung für eine Zerfallstablette nach einem der Ansprüche 1 bis 6, umfassend:
Einblasen von Heißluft in einen unteren Teil des Pulvers, das aus anderen Komponenten als der mikrofibrillierten Cellulose besteht, in einem Wirbelschichtgranulator; und
Sprühen einer Dispersionsflüssigkeit (Aufschlämmung) der mikrofibrillierten Zellulose auf einen oberen Teil des Pulvers, während das Pulver fluidisiert wird.

## Revendications

1. Composition pour comprimé désintégrant, comprenant un composant désintégrant constitué uniquement de cellulose microfibrillée, et un ingrédient actif, dans laquelle la composition ne comprend aucun agent désintégrant autre que la cellulose microfibrillée.

2. Composition pour comprimé désintégrant selon la revendication 1, comprenant en outre un excipient.

3. Composition pour comprimé désintégrant selon la revendication 2, comprenant des sucres ou des alcools de sucre comme excipient.

4. Composition pour comprimé désintégrant selon la revendication 1, constituée de la cellulose microfibrillée et de l'ingrédient actif.

5. Composition pour comprimé désintégrant selon l'une quelconque des revendications 1 à 4, dans laquelle la cellulose microfibrillée a une longueur moyenne de fibre de 0,01 à 2 mm et un diamètre moyen de fibre de 0,001 à 1 µm.

6. Composition pour comprimé désintégrant selon l'une quelconque des revendications 1 à 5, comprenant 80 à 90 % en poids de l'ingrédient actif.

7. Comprimé désintégrant pour produits pharmaceutiques ou alimentaires, comprenant la composition pour comprimé désintégrant selon l'une quelconque des revendications 1 à 6, dans lequel le comprimé ne comprend aucun agent désintégrant autre que la cellulose microfibrillée.

8. Comprimé désintégrant selon la revendication 7, qui a une dureté de comprimé de 10 à 200 (N), et un temps de désintégration dans l'eau de 6 min ou moins, et/ou [le temps de désintégration dans l'eau (D)]/[la dureté du comprimé (H)] est de 30 (sec/N) ou moins.

9. Procédé de production de la composition pour comprimé désintégrant selon l'une quelconque des revendications 1 à 6, comprenant :
l'injection d'air chaud dans une partie inférieure de poudre constituée de composants autres que la cellulose microfibrillée dans un granulateur à lit fluidisé ; et
la pulvérisation d'un liquide de dispersion (boue) de la cellulose microfibrillée sur une partie supérieure de la poudre lors de la fluidisation de la poudre.
